# EUROPEAN PATENT SPECIFICATION

(11) **EP 3 016 710 B1**
(45) Date of publication and mention of the grant of the patent: **08.04.2020**
(21) Application number: 14742123.4
(22) Date of filing: 03.07.2014
(51) Int. Cl.: A61M 25/09

(54) **GUIDEWIRE**
FÜHRUNGSDRAHT
FIL-GUIDE

(30) Priority: 03.07.2013 US 201361842692 P
(43) Date of publication of application: 11.05.2016
(73) Proprietor: Boston Scientific Scimed, Inc., Maple Grove, MN 55311 (US)
(72) Inventor: ABNER, William, Rutland, MA 01543 (US); WALAK, Steven E., Natick, MA 01760 (US); GAFFNEY, Brian, Rutland, MA 01543 (US); STORBECK, Gene T., Franklin, MA 02038 (US)
(74) Representative: Vossius & Partner Patentanwälte Rechtsanwälte mbB
(86) International application number: PCT/US2014/045385
(87) International publication number: WO 2015/003121

(56) References cited:
- EP-A1- 1 388 349
- EP-A2- 1 348 461
- WO-A1-97/32625
- US-A- 5 938 623
- US-A1- 2001 009 980

## Description

### Field of the Disclosure

Embodiments of the present disclosure generally relate to medical devices and associated methods of use. In particular, embodiments of the present disclosure relate to guidewires for use in medical procedures.

### Background of the Disclosure

EP 1 348 461 A2 describes a guidewire that includes a linear core member, a resin layer formed to cover a main body portion of the core member, and a contrasting portion formed to cover a distal end portion of the core member.

WO 97/32625 A1 describes an intravascular guidewire. The guidewire has an elongated selectively formable core and a plastic jacket including proximal and distal jacket portions. US 5 938 623 A describes a guide wire with an adjustable stiffness. A flexible coil is secured to a distal extremity of a core wire of the guide wire and extends over a portion of the core wire having a reduced diameter.

US 2001/009980 A1 describes a guidewire with multiple polymer jackets over distal and intermediate core sections.

EP 1 388 349 A1 describes a guide wire including a wire member having a first wire disposed on a distal side of the guide wire and a second wire disposed on a proximal side from the first wire. A cover layer is formed on an outer peripheral surface of a wire member.

During certain medical procedures, a guidewire may be used to access a target area of a patient's body. Accessing the target area with the guidewire may include navigating the guidewire through tortuous passages and/or openings of the patient's body. Once in position, the guidewire may facilitate movement of another medical device to the target area. For instance, the guidewire may be inserted through the patient's body to the target area, and may act as a rail or support to facilitate delivery of a catheter, stent, cannula, endoscope, cutting device, retrieval basket, or the like, to the target area.

In one type of procedure, a guidewire can be used to access a bile duct in the patient's body. Next, a medical device may be advanced over the inserted guidewire and toward the bile duct, using the inserted guidewire as a rail. The characteristics of the guidewire may have an effect on how efficiently these, and other procedures, can be performed. For example, if the guidewire is too thick, it may be difficult to gain entry into the bile duct. If the guidewire is too thin, it may not be rigid enough to support the medical device, and may be easily bent so as to withdraw from the bile duct.

Therefore, guidewires having performance-enhancing characteristics are desired.

### SUMMARY OF THE DISCLOSURE

The present invention is defined by the subject-matter of its claims. In the following summary, the term "width" is to be understood as relating to an outer diameter. According to an embodiment of the present disclosure, a guidewire for performing medical procedures may include a central core. The central core may include a first region having a first width and a distal surface. The central core may also include a second region distal to the first region. The second region may have a second width different than the first width. The second region may also have a proximal surface. At least a portion of the distal surface of the first region may be angled relative to a portion of the proximal surface of the second region. The guidewire may also include a first covering surrounding the first region. The first covering and the first region may form a first covered region having a third width. The guidewire may also include a second covering surrounding the second region. The second covering and the second region may form a second covered region having a fourth width. The third width and the fourth width may be substantially equal.

According to another embodiment of the present disclosure, a guidewire for performing medical procedures may include a central core. The central core may include a first region having a first width and a distal surface. The central core may also include a second region distal to the first region. The second region may have a second width different than the first width. The second region may also have a proximal surface. At least a portion of the distal surface of the first region may be angled relative to a portion of the proximal surface of the second region. The central core may also include a third region distal to the second region. The third region may have a third width different than the second width. The central core may also include a fourth region distal to the third region. The fourth region may have a fourth width different than the third width. The guidewire may also include a first covering surrounding the first region. The first covering and the first region may form a first covered region having a fifth width. The guidewire may also include a second covering surrounding the second region and at least a portion of the third region. The second covering and the second region may form a second covered region having a sixth width. The fifth width and the sixth width may be substantially equal. The guidewire may also include a third covering surrounding the fourth region and at least a portion of the third region.

According to another embodiment of the present disclosure, a method of using a guidewire to introduce a medical device to a target area in a patient's body may include inserting the guidewire to the target area through the patient's body. The method may also include inserting a distal tip of the guidewire into an opening at the target area of the patient's body. The method may also include inserting a tapered region of the guidewire, proximal to the distal tip, into the opening. The method may also include identifying when a proximal end of the tapered region has reached the opening. The method may also include advancing a medical device over the guidewire to the target area while the distal tip and the tapered region remain in the opening, once the proximal end of the tapered region has reached the opening.

Additional characteristics, features, and advantages of the described embodiments will be set forth in part in the description that follows, and in part will be apparent from the description, or may be learned by practicing the disclosure. The disclosed subject matter can be realized and attained by way of the elements and combinations particularly pointed out in the appended claims.

It is to be understood that both the foregoing general description and the following detailed description are exemplary and explanatory only, and are not restrictive of the described embodiments, as claimed.

### BRIEF DESCRIPTION OF THE DRAWINGS

The accompanying drawings, which are incorporated in, and constitute a part of this specification, illustrate exemplary embodiments of the present disclosure and, together with the description, serve to explain the principles of the disclosure.
FIG. 1 is an illustration of a system, including an exemplary guidewire, placed inside the body of a patient, in accordance with principles of the disclosure;
FIG. 2 is a cross-sectional view of the guidewire of FIG. 1, in accordance with principles of the disclosure;
FIG. 2A is an enlarged view of an area "A" of FIG. 2, in accordance with principles of the disclosure;
FIG. 2B is a close-up view of a joint region of the guidewire of FIG. 1, in accordance with principles of the present disclosure.
FIG. 3A illustrates exemplary dimensions of regions of the guidewire of FIG. 2, in accordance with principles of the disclosure;
FIG. 3B illustrates exemplary amounts of a tungsten filler used in the guidewire of FIG. 2, in accordance with principles of the disclosure;
FIG. 3C illustrates exemplary materials for a coating and exemplary coating thicknesses for the guidewire of FIG. 2, in accordance with principles of the disclosure; and
FIG. 3D illustrates exemplary materials for a covering, and exemplary ways to construct coverings for the guidewire of FIG. 2, in accordance with principles of the disclosure.

### DESCRIPTION OF THE EMBODIMENTS

Reference will now be made in detail to embodiments of the present disclosure, examples of which are illustrated in the accompanying drawings. Wherever possible, the same reference numbers will be used throughout the drawings to refer to the same or like parts.

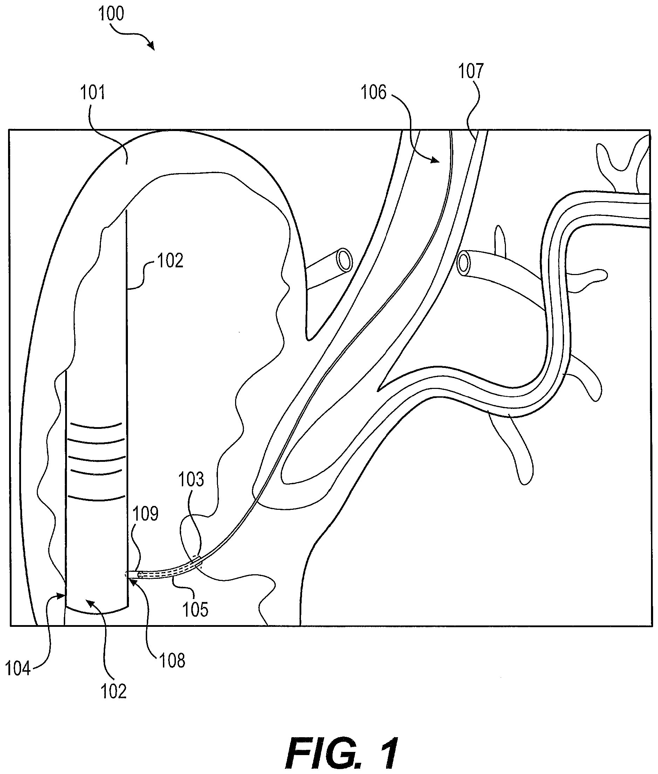

FIG. 1 is a schematic view of a system 100, inserted into a region of a patient's body including, for example, a patient's duodenum 101. The system 100 may include a longitudinal shaft 102, such as a sheath, catheter, endoscope, or the like, with the longitudinal shaft 102 terminating at a distal end 104. The shaft 102 may include a central lumen (not shown) configured to receive one or more medical devices. The shaft 102 may also include a side opening 108 in communication with the central lumen. An elevator 109 may be positioned at the side opening 108. When a medical device exits from the side opening 108, the elevator 109 may be moved (e.g., raised, lowered, rotated, and/or tilted) to facilitate positioning of the exiting medical device.

A guidewire 106 may be introduced into a central lumen of a cannula or sphincterotome 105. The guidewire 106 and sphincterotome 105 may be inserted into a proximal portion (not shown) of the shaft 102, and may be advanced through the central lumen of the shaft 102, toward the side opening 108. The sphincterotome 105 and the guidewire 106 may emerge from the opening 108, and may extend through or otherwise engage the elevator 109. As the sphincterotome 105 and the guidewire 106 extend from the opening 108, the elevator 109 may be moved to facilitate positioning of the sphincterotome 105 and the guidewire 106. For example, the elevator 109 may be tilted to redirect the sphincterotome 105 and the guidewire 106 to align with an opening, such as the patient's papilla 103. As the sphincterotome 105 and the guidewire 106 extend further out from the opening 108, portions of the guidewire 106 may be extended from the sphincterotome 105 so that those portions of the guidewire 106 may enter the papilla 103, and may proceed into the patient's bile duct 107. The sphincterotome 105 may be removed after the guidewire 106 has been inserted, by being withdrawn proximally from the guidewire 106. The guidewire 106 may act as a rail or support for introducing one or more other medical devices, such as a catheter, cannula, stent, retrieval basket, snare, or the like, to a target area of the patient's body. Further details of the guidewire 106 will be described in conjunction with subsequent figures.

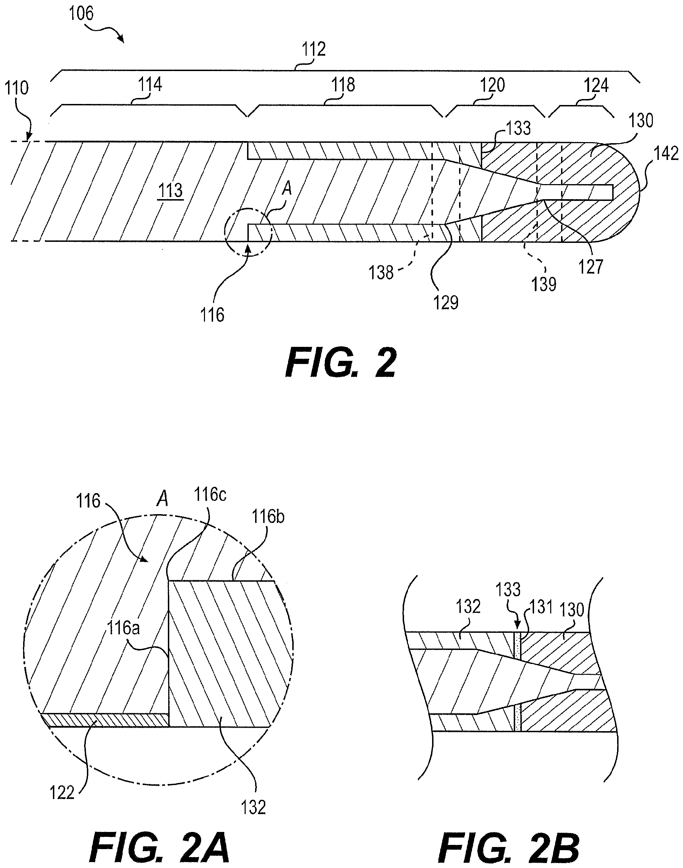

FIG. 2 shows a cross-sectional side view of the guidewire 106 of FIG. 1. The guidewire 106 may include an elongated member 110. The elongated member 110 may be advanced to the target area through tortuous anatomy or a narrow body lumen, such as through a portion of a patient's gastrointestinal tract. The elongated member 110 may include an end portion 112. The end portion 112 may travel through a proximal opening (not shown) of the shaft 102, through the central lumen (not shown) of the shaft 102, out the opening 108 of the shaft 102 (see FIG. 1), and to a target area.

In one embodiment, the end portion 112 of the elongated member 110 may include a wire core 113. The core 113 may have a first region 114, a second region 118, a third region 120, and a fourth region 124. In one embodiment, each of the regions may have a diameter different than diameters of the other regions. The core 113 may be made of, for example, any suitable biocompatible material, which may include, but is not limited to, metals, metal alloys, polymers, reinforced polymer composites, or the like. In some embodiments, the core 113 may be formed from one or more shape memory alloys, such as nitinol.

The first region 114 may extend to a proximal end (not shown) of the guidewire 106. A first covering 122 (FIG. 2A) may be applied over the first region 114. The first covering 122 may be substantially thin to minimize the incremental increase in the outer diameter of the first region 114. In one embodiment, the first covering 122 may be between approximately 0.0001 to 0.0010 inches (0.0025 to 0.0254mm) thick. In another embodiment, the first covering 122 may be between approximately 0.0002 to 0.0004 inches (0.0051 to 0.0102mm) thick. In yet another embodiment, the first covering 122 may be approximately 0.0004 inches (0.0102mm) thick. In yet another embodiment, the first covering 122 may be approximately 0.0007 inches (0.0178mm) thick. At the proximal end of the guidewire 106, the first covering 122 may include a modified surface finish for enhanced grip and control when grasped by a user.

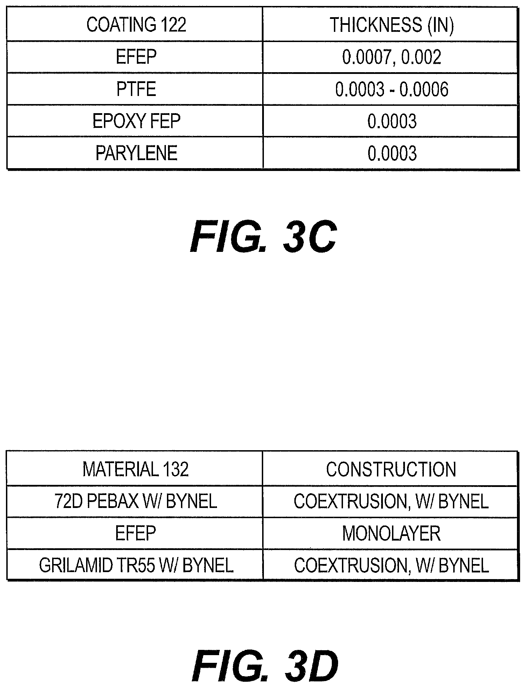

The first covering 122 may be made of parylene, polyimide, polytetrafluoroethylene (PTFE), a PTFE blend, fluorinated ethylene propylene (FEP), a FEP blend, epoxy FEP, perfluorinated ethylene-propylene copolymer (EFEP), an EFEP blend, or any other suitable coating. These types of coatings may be low-friction or lubricious, reducing impediments to the sliding of medical devices over the guidewire 106, while also helping to preserve the straightness of the guidewire 106. In one embodiment, the first covering 122 may be a PTFE spray coating. Exemplary materials for first covering 122, and exemplary thicknesses for first covering 122, are illustrated in FIG. 3C.

It is contemplated that in one embodiment, the first covering 122 may include a plurality of layers (not shown). Each of the layers may extend circumferentially around the core 113 in the first region 114. The layers may have substantially equal thicknesses. However, it is also contemplated that one or more of the layers may have a thickness different than a thickness of another of the one or more layers.

The second region 118 extends distally from the first region 114. The second region 118 may have a different diameter than the first region 114. For example, the second region 118 may have a smaller diameter than the first region 114. It is also contemplated that one or more portions of the second region 118 may have a smaller diameter than the first region 114, and one or more portions of the second region 118 may have a larger diameter than the first region 114.

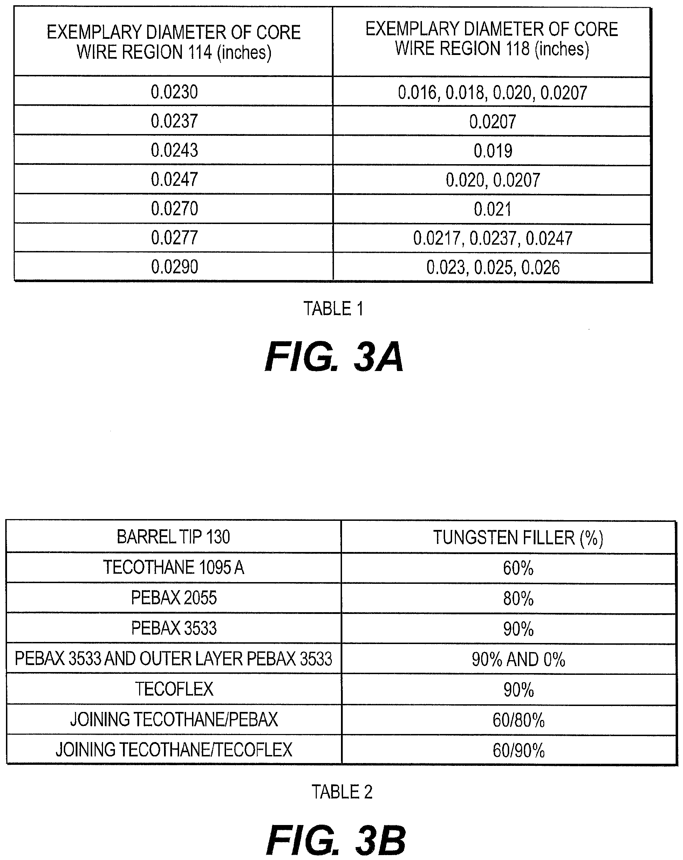

Exemplary diameters for portions of the core 113, such as the first region 114 and the second region 118, are shown in TABLE 1 (FIG. 3A). It is contemplated that any combination of diameters in Table 1 may be used to make the guidewire 106. It is also contemplated that other diameters may be used. For example, it is contemplated that the guidewire 106 may have a diameter in a range of approximately 0.025 inches to 0.035 inches (0.635 to 0.889mm). It is also contemplated that the guidewire 106 may have a diameter of approximately 0.014, 0.018, 0.021, or 0.038 inches (0.3556, 0.4572, 0.5334, 0.9652mm). The diameter of the core 113 and any coverings thereon may be selected/adjusted to produce a guidewire with any desired diameter.

The diameter of the first region 114 may be substantially constant from one end of the first region 114 to the other. The diameter of the second region 118 may also be substantially constant from one end of the second region 118 to the other. When the second region 118 has a smaller diameter than the first region 114, the smaller diameter of the second region 118 may give that region more flexibility than the first region 114, which may facilitate navigation of the distal portion 112 through body openings and tortuous anatomy. Further, the smaller diameter of the second region 118 may provide space for a second covering 132 to encompass the second region 118, without having the second covering 132 extend radially beyond an outer diameter defined by the first covering 122. As such, the second covering 132 may have a different thickness than the first covering 122. The second covering 132 will be described in more detail below.

The first region 114 and the second region 118 may meet at a transition region 116. As shown in FIG. 2A, the transition region 116 may include a surface 116a, defined by a distal end face of the first region 114, and a surface 116b forming part of a proximal end portion of the second region 118. The surfaces 116a and 116b may be angled relative to one another. For example, in one embodiment, the angle between the surfaces 116a and 116b may be between approximately 45 degrees and approximately 135 degrees. In another embodiment, the angle between the surfaces 116a and 116b may be between approximately 70 degrees and approximately 110 degrees. In another embodiment, the angle between the surfaces 116a and 116b may be substantially perpendicular. The junction 116c between the surfaces 116a and 116b may include a sharp corner. It is also contemplated that the junction 116c may include a curved portion, with the size of the curved portion being minimized to ensure that the proximal end portion of the second covering 132 is not pushed radially outward by the junction 116c. The transition region 116 may extend circumferentially around the elongated member 110.

It is contemplated that the transition region 116 may be located between approximately 15 cm and approximately 90 cm from a distal end of the fourth region 124. In another embodiment, the transition region 116 may be located between approximately 50 cm and approximately 70 cm from the distal end of the fourth region 124. In another embodiment, the transition region 116 may be located approximately 60 cm from the distal end of the fourth region 124. In yet another embodiment, the transition region 116 may be located between approximately 35 cm and approximately 70 cm from the distal end of the fourth region 124. For example, the transition region 116 may be located at approximately 25 cm from the distal end of the fourth region 124.

It is also contemplated that the transition region 116 may include a plurality of small transition regions, forming a plurality of steps (not shown). Each of those steps may include surfaces similar to the surfaces 116a and 116b, having characteristics similar to those of the surfaces 116a and 116b described above.

A third region 120 may extend distally from a distal end of the second region 118, to a proximal end of the fourth region 124. A diameter of the core 113 in the third region 120 may differ from a diameter of the core 113 in the second region 118, and/or may vary in a distal direction. For example, the diameter of the core 113 in the third region 120 may decrease in the distal direction, such that the third region 120 may form a taper between the distal end of the second region 118 and the proximal end of the fourth region 124. The taper may include a smooth, sloped surface, and/or a sloped surface formed by a plurality of steps. It is also contemplated that the diameter of the core 113 in the third region 120 may decrease in the distal direction over a first portion, and increase or remain constant in the distal direction over a second portion.

It is contemplated that the distal end of the third region 120 may be approximately 10 cm from a distal end of the fourth region 124. The core 113 in the transition region 116 is steeper than the third region 120, in that the transition region 116 undergoes its change in diameter over a shorter distance than the distance in which the third region 120 undergoes its change in diameter.

The fourth region 124 extends distally from the distal end of the third region 120. The diameter of the core 113 in the fourth region 124 may be substantially constant from one side of the fourth region 124 to the other, and/or may be less than the diameter of the core 113 in the third region 120. It is also contemplated that the diameter of the core 113 in the fourth region 124 may vary. For example, the diameter may increase over a portion of the fourth region 124, and/or may decrease over a portion of the fourth region 124.

The dimensions of the fourth region 124 may facilitate entry of the fourth region 124 into a body opening, such as the papilla 103. The dimensions of the core 113 in the fourth region 124 may also be configured such that when a distal end of the fourth region 124 encounters an obstruction or constriction in a body lumen, such as in the bile duct 107, the proximal end of the fourth region 124 may bend, initiating formation of a loop. To form the loop, the distal portion 112 may bend back on itself to form a "U" shape.

An area 127 lies where the proximal end of the fourth region 124 meets the distal end of the third region 120, and marks the location where looping may initiate. Proximal to the area 127, the diameter of the core 113 may begin to increase in a proximal direction, giving the third region 120 its taper. As the diameter of the core 113 increases, its strength and/or stiffness may also increase.

An area 129 lies where the proximal end of the third region 120 meets the distal end of the second region 118. During use, once the distal portion 112 has been inserted into an opening far enough, such that the area 129 is at the opening (and the fourth region 124 and third region 120 are through the opening), the inserted length of the distal portion 112 (e.g., the length of the fourth region 124 and third region 120) may have sufficient strength and/or stiffness to allow introduction of a medical device over the guidewire 106, and to the opening, without pulling the inserted length out of the opening due to bending of the inserted length.

The sheath or second covering 132 may encompass the second region 118 and at least a portion of the third region 120. The second covering 132 may have a thickness such that an outer diameter of the second covering 132 is substantially equal to an outer diameter defined by the first covering 122. For example, the thickness of the second covering 132 may be in the range of 0.002 to 0.003 inches (0.051 to 0.762mm). Further, any seam formed between the proximal end of the second covering 132 and the first covering 122 and/or the surface 116a, may have a width of 0.005 inches (0.127mm) or less. Furthermore, the second covering 132 may have a black and yellow spiral color, or any other suitable outward appearance, to enhance its visibility. The second covering 132 may be formed from a suitable flexible, lubricious, and/or biocompatible material. Examples of materials may include one or more of PTFE, FEP, EFEP, Tecothane, a polyether block amide like PEBAX, such as 72D PEBAX, a polyamide material like Grilamid TR55, combinations of polymers, and/or any other suitable materials. In some embodiments, the second covering 132 may be coated with a hydrophilic coating, to enhance lubricity. In some embodiments, second covering 132 may be lubricious enough that a hydrophilic coating may be omitted.

It is contemplated that in one embodiment, the second covering 132 may include a plurality of layers (not shown). Each of the layers may extend circumferentially around the core 113 in the second region 118. The layers may have substantially equal thicknesses. However, it is also contemplated that one or more of the layers may have a thickness different than a thickness of another of the one or more layers. It is also contemplated that one or more of the layers may extend continuously across both the first region 114 and the second region 118. One or more layers of the second covering 132 may increase the bond strength between one or more other layers of the second covering 132 and the core wire 113.

In one contemplated embodiment, the second covering 132 may be made of 72D PEBAX, without tungsten loading, the 72D PEBAX being coextruded with an adhesive resin like BYNEL. In another contemplated embodiment, the second covering 132 may be made of Grilamid TR55 coextruded with BYNEL. In yet another contemplated embodiment, the second covering 132 may be made of a monolayer of reflowed EFEP. Exemplary materials for second covering 132, and exemplary construction processes for second covering 132, are illustrated in FIG. 3D.

A visual indicator 138 may be applied to the guidewire 106 to provide an indication of the location of the area 129. The visual indicator 138 may be in the form of a strip, band, or a coil, or any other suitable form. Additionally or alternatively, the visual indicator 138 may include printed ink. It is also contemplated that the visual indicator 138 may be radiopaque for viewing under fluoroscopy. For example, the visual indicator 138 may include one or more of tungsten, platinum, gold, tantalum, palladium, salts thereof, alloys thereof, or the like It is also contemplated that the visual indicator 138 may be visible to the naked eye.

The visual indicator 138 may be positioned on the second covering 132, at least partially round the second covering 132, at least partially embedded within the second covering 132, or positioned around the area 129, underneath the second covering 132. The visual indicator 138 may be centered over the area 129. For example, a center of the visual indicator 138 may be located a distance of approximately 10 cm from the distal end of the fourth region 124. Alternatively, an edge of the visual indicator 138 may be positioned over the area 129. It is also contemplated that the visual indicator 138 may be at least partially raised from a surface of the second covering 132, or may be flush with the surface of the second covering 132.

The fourth region 124, and at least a portion of the third region 120, may be encompassed by a substantially constant diameter third covering 130. The third covering 130 (FIG. 2) may be applied over the core 113 in at least one of the third region 120 and the fourth region 124. The third covering 130 may engage surfaces of the core 113, and a distal end of the second covering 132. A joint 133 may be formed at the distalmost end of the second covering 132 and the proximalmost end of the third covering 133. Material in the third covering 130 may be reflowed with or blended with material from the second covering 132 to couple the second and third coverings 130 and 132. The transition region between the second covering 132 and the third covering 130 may extend around the third region 120. For example, the transition region may extend circumferentially around the third region 120 about a middle portion of the taper. It is contemplated that characteristics (e.g., shape and thickness) of the third covering 130 may be selected such that the diameter of the guidewire 106 may be substantially constant along each portion of the distal portion 112.

The third covering 130 may extend distally beyond the core 113 to help protect tissue from being punctured or otherwise damaged by the fourth region 124, during insertion of the distal portion 112 into and/or through the patient's body. The third covering 130 may be formed from one or more of, a polyurethane like TECOTHANE1095 A, PEBAX 2533, PEBAX 2055, PEBAX 3533, an aliphatic polyether polyurethane like TECOFLEX, joined TECOTHANE/PEBAX, or jointed TECOTHANE/TECOFLEX. The material of the third covering 130 may also include a Tungsten filler to increase the radiopacity of the third covering 130, making the distal tip of the guidewire 106 visible via fluoroscopy. Exemplary amounts of filler are shown in TABLE 2 (FIG. 3B). The radiopacity of the visual indicator 138 may be different from the radiopacity of the third covering 130, so they can be distinguished from each other under fluoroscopy.

It is contemplated that in one embodiment, the third covering 130 may include a plurality of layers (not shown). Each of the layers may extend circumferentially around the core 113 in the third region 120 and/or the fourth region 124. The layers may have substantially equal thicknesses. However, it is also contemplated that one or more of the layers may have a thickness different than a thickness of another of the one or more layers. It is also contemplated that one or more of the layers may extend continuously across two or more of the first region 114, second region 118, third region 120, and fourth region 124. In one contemplated embodiment, the third covering 130 may including a radially inner layer of BYNEL; an intermediate layer of TECOTHANE, TECOFLEX, and/or PEBAX loaded with Tungsten filler; and a radially outer layer of PEBAX, without Tungsten filler, for its dielectric properties. The BYNEL layer may be between approximately 0.001 to 0.002 inches (0.0254 to 0.051mm) thick. The outer layer of PEBAX may be approximately 0.001 inches (0.0254mm) thick.

When the second covering 132 is made of one type of material and the third covering 130 is made of another type of material, a ring or sleeve 131 (FIG. 2B) may be provided at the joint 133. The ring 131 may help bond the second covering 132 and the third covering 130. For example, the ring 131 may act as a reflow binder to bond with the second covering 132 and the third covering 130 during a reflowing step. Without the ring 131, the second covering 132 may not reflow and bond properly to the third covering 130.

In one embodiment, the ring 131 may be made of a polyether block amide material like VESTAMID Care PEBA ME-B. The ring 131 may be between approximately 0.1 cm and 1.0 cm wide. The second covering 132 may be made of EFEP, and the third covering 130 may be made of PEBAX. In the absence of the ring 131, the EFEP forming the second covering 132 may flow under the PEBAX forming the third covering 130, lifting the third covering 130 off of the core 113 and creating an undesirable lip or other discontinuity at or near the joint 133. With the ring 131 at the joint 133 during the reflowing step, the EFEP forming the second covering 132 may bond with the PEBA forming the ring 131, and the PEBAX forming the third covering 130 may bond with the PEBA forming the ring 131, thus bonding the second covering 132 with the third covering 130. It is contemplated that the ring 131 may be omitted when the second covering 132 is formed of PTFE or FEP, since PTFE and FEP may create a better bond with PEBAX than EFEP when reflowed.

A visual indicator 139 may be applied to the guidewire 106 to provide an indication of the location of the area 127. The visual indicator 139 may be similar to the visual indicator 138. The visual indicator 139 may differ in that the radiopacity of the visual indicator 139 may be different from that of the third covering 130 and/or the visual indicator 138, so that the visual indicator 139 may be distinguishable from the third covering 130 and/or the visual indicator 138. The visual indicator 139 may be positioned around the third covering 130, embedded within the third covering 130, or positioned around the area 127 underneath the third covering 130. The visual indicator 139 may be centered over the area 127. Alternatively, an edge of the visual indicator 139 may be positioned at the area 127.

Aspects of the guidewire 106 may enhance the performance of guidewire 106 when used in a procedure. The length of guidewire 106 may allow a proximal portion of the guidewire 106 to extend through the shaft 102, while also allowing a distal portion of the guidewire 106 to extend distally out of the shaft 102 and into a target area, even when guidewire 106 travels through a subject's anatomy. Reducing the diameter of the core 113 in one or more of the regions 118, 120, and 124 may create thin bendable regions that have less stress and strain in their bends than in the region 114, making navigation through tortuous passages easier. The thick diameter of the region 114 may help improve pushability of the guidewire 106, enhance the tactile feel of the device in the hand of the user, and/or help transmit rotational forces from the proximal portion of the guidewire 106 to the distal portion of the guide wire 106.

Covering the core 113 with one or more of the coverings 122, 130, and 132 may help with insertion of the guidewire 106 due to their lubricity. Their lubricity may also help a user rotate the distal portion of the guidewire 106 in a controllable way. To rotate the distal portion of the guidewire 106, the user may rotate a proximal portion of the guidewire 106. If the distal portion of the guidewire 106 sticks to one or more surfaces in a subject's body, it may not rotate as the proximal portion of the guidewire 106 is rotated. When a sufficient twisting force has been imparted to the proximal portion of the guidewire 106, and/or an intermediate portion of the guidewire 106, the twisting force may overcome the sticking force (e.g., friction) between the distal portion of the guidewire 106 and the body surface, causing the distal portion of the guidewire 106 to rotate abruptly or whip. Because of the whipping, the user may not be able to position the distal portion of the guidewire 106 with enough accuracy to perform certain procedures.

From the user's perspective, it may be more desirable for there to be a one-to-one relationship between rotation of the proximal portion of the guidewire 106 and rotation of the distal portion of the guidewire 106. The lubricity of coverings 122, 130, and 132 may enhance the user's control by reducing sticking/friction between the guidewire 106 and body surfaces, leading to less whipping and a closer relationship between proximal portion rotation and distal portion rotation for the guidewire 106. The reduced diameter portions of core 113 may also reduce whipping by ensuring that there is less stress and strain built up in bent portions of the guidewire 106 when in use, since stress and strain can hinder rotation.

Coverings 122, 130, and/or 132 may also provide dielectric resistance for accessory products, like the sphincterotome 105, reducing the likelihood of capacitive coupling developing between the sphincterotome 105 and the core 113, and of a direct short developing between the sphincterotome 105 and the core 113. Materials for the coverings 122, 130, and 132 may also enhance guidewire performance in other ways. For example, an EFEP covering may be highly lubricious, while being thinner than other coverings. When the EFEP covering is on a bent region of the guidewire 106, the thinness of the covering may reduce stress and strain at the bend, and the lubricity of the covering may reduce sticking, leading to enhanced rotational control for the user. An FEP covering may bond well with core 113. A 72D PEBAX covering may be hard, receptive to co-extrusion with BYNEL for bonding to core 113, and receptive to receiving a hydrophilic coating for added lubricity. A Grilamid TR55 coating may have higher impact strength and/or durability than the other coatings, and may extrude well with BYNEL for bonding to core 113, making the coating desirable when the guidewire 106 is aggressively bent by, for example, the elevator 109.

A method of accessing a body opening, such as a patient's papilla 103, and treating a target area, such as the patient's bile duct 106, may include inserting the shaft 102 into a passageway in a patient's body, such as the patient's duodenum 101. The distal end 104 of the shaft 102 may be positioned near the papilla 103, with the opening 108 and elevator 109 turned towards the papilla 103.

The guidewire 106 may be inserted through the central lumen of the shaft 102 before the shaft 102 is inserted into the patient, during insertion of the shaft 102, or after insertion of the shaft 102. The guidewire 106 may be pushed out of the opening 108, and the elevator 109 may be actuated to guide the guidewire 106 toward the papilla 103.

The distal portion 112 of the guidewire 106 may approach the papilla 103. The guidewire distal tip 142, which may include a rounded distal end of the third covering 130, may reach the papilla 103, and its relatively low compliance (compared to other portions of the guidewire 106) may help facilitate its entry into the papilla 103. The distal portion 112 may be pushed through the papilla 103 until the visual indicator 138 reaches the papilla 103, indicating that the area 129 has reached the papilla 103. The user may be able to view the visual indicator 138 reaching the papilla 103 via fluoroscopy. Once this happens, the user may be assured that the length of the distal portion 112 inserted through the papilla 103 has a sufficient strength and/or stiffness, such that medical devices may travel over the guidewire 106, using the guidewire 106 as a rail, without causing the inserted length to be bent severely enough to be pulled out of the papilla 103.

The guidewire 106 may be inserted even further through the papilla 103, into, for example, the bile duct 107. Using fluoroscopy, the user may track movement of the guidewire distal tip 142, providing the user with an indication of the depth to which the guidewire 106 has been inserted. If, in the bile duct 107, the guidewire distal tip 142 contacts an obstruction or constriction, such as a stone (not shown) in the bile duct 107, the guidewire distal tip 142 may engage the stone. The fourth region 124 may, at the area 127, begin bending to form a loop. Using fluoroscopy, the user may identify that bending has occurred, and based thereon, may identify an approximate location of the stone in the bile duct 107. If no bending occurs, it may indicate that the bile duct 107 is clear of obstructions or constrictions.

If an obstruction, such as a stone, is found, a medical device, such as a retrieval basket, may be inserted over and along the guidewire 106, using the guidewire 106 as a rail, to deliver the retrieval basket to the stone. The retrieval basket may be actuated to capture the stone, after which the retrieval basket may be withdrawn from the guidewire 106.

Embodiments of the present disclosure may be applicable to various and different medical or non-medical procedures. In addition, certain aspects of the aforementioned embodiments may be selectively used in collaboration, or removed, during practice, without departing from the scope of the disclosure.

Other embodiments of the present disclosure will be apparent to those skilled in the art from consideration of the specification and practice of the embodiments disclosed herein. It is intended that the specification and examples be considered as exemplary only, with a true scope of the disclosure being indicated by the following claims.

## Claims

1. A guidewire (106) for performing medical procedures, the guidewire (106) comprising:
a central core (113), including:
a first region (114) having a first outer diameter and a distal surface (116a), the distal surface (116a) being a distal end face of the first region (114), and
a second region (118) distal to the first region (114), the second region (118) having a second outer diameter different than the first outer diameter, and the second region (118) having a proximal surface (116b), wherein at least a portion of the distal surface (116a) of the first region (114) is angled relative to a portion of the proximal surface of the second region (118);
a first covering (122) surrounding the first region (114), the first covering (122) and the first region (114) forming a first covered region, the first covering (122) having a distal end at the distal end face of the first region (114), and the first covered region having a third outer diameter; and
a second covering (132) surrounding the second region (118), the second covering (132) and the second region (118) forming a second covered region, and the second covered region having a fourth outer diameter, wherein the third outer diameter and the fourth outer diameter are substantially equal.

2. The guidewire (106) of claim 1, wherein the proximal surface (116b) of the second region (118) is a radially outer surface of the second region (118).

3. The guidewire (106) of claim 2, wherein the distal end face is substantially perpendicular to the radially outer surface.

4. The guidewire (106) of claim 2, wherein the distal end face and the radially outer surface meet at a curved surface.

5. The guidewire (106) of claim 1, wherein the central core (113) further includes a third region (120) distal to the second region (118), the third region (120) having a fifth outer diameter different than the second outer diameter.

6. The guidewire (106) of claim 5, wherein the central core (113) further includes a fourth region (124) distal to the third region (120), the fourth region (124) having a sixth outer diameter different than the fifth outer diameter, and the fourth region (124) including a distal tip (142) of the central core (113).

7. The guidewire (106) of claim 1, wherein
the central core (113) further comprises
a third region (120) distal to the second region (118), the third region (120) having a fifth outer diameter different than the second outer diameter, and
a fourth region (124) distal to the third region (120), the fourth region (124) having a sixth outer diameter different than the fifth outer diameter;
wherein the third region (120) is tapered from the second outer diameter to the sixth outer diameter,
wherein the second covering (132) surrounds at least a portion of the third region (120),
wherein the guidewire (106) further comprises a third covering (130) surrounding the fourth region (124) and at least a portion of the third region (120), wherein a joint between a distal-most end of the second covering (132) and a proximal-most end of the third covering (130) is located along the tapered third region.

8. The guidewire (106) of claim 7, wherein the second covering (132) is a sheath.

9. The guidewire (106) of claim 7, wherein the second covering (132) is made of a first material, the third covering (130) is made of a second material different from the first material, and the guidewire (106) includes a ring (131) at a junction (133) between the second covering (132) and the third covering (130), the ring (131) being made of a third material different from the first material and second material.

10. The guidewire (106) of claim 7, wherein a wall thickness of a portion of the second covering (132) surrounding the third portion decreases in a distal direction.

11. The guidewire (106) of claim 7, wherein the first region (114), the second region (118), and the fourth region (124) are each substantially cylindrical.

12. The guidewire (106) of claim 7, further including a radiopaque visual indicator (138) applied to the second covering (132) where a distal end of the second region (118) meets a proximal end of the third region (120).

13. The guidewire (108) of claim 7, further including a radiopaque visual indicator (138) applied to the third covering (130).

14. The guide wire (106) of claim 13, wherein the radiopaque visual indicator (138) is applied to the third covering (130) where a distal end of the third region (120) meets a proximal end of the fourth region (124).

## Patentansprüche

1. Führungsdraht (106) zum Durchführen von medizinischen Prozeduren, wobei der Führungsdraht (106) aufweist:
einen mittleren Kern (113), der aufweist:
einen ersten Bereich (114), der einen ersten Außendurchmesser und eine distale Fläche (116a) aufweist, wobei die distale Fläche (116a) eine distale Endfläche des ersten Bereichs (114) ist, und
einen zweiten Bereich (118) distal vom ersten Bereich (114), wobei der zweite Bereich (118) einen zweiten Außendurchmesser aufweist, der sich vom ersten Außendurchmesser unterscheidet, und wobei der zweite Bereich (118) eine proximale Oberfläche (116b) aufweist, wobei mindestens ein Abschnitt der distalen Fläche (116a) des ersten Bereichs (114) relativ zu einem Abschnitt der proximalen Fläche des zweiten Bereich (118) angewinkelt ist;
eine erste Abdeckung (122), die den ersten Bereich (114) umgibt, wobei die erste Abdeckung (122) und der erste Bereich (114) einen ersten abgedeckten Bereich bilden, wobei die erste Abdeckung (122) ein distales Ende an der distalen Endfläche des ersten Bereichs (114) aufweist, und wobei der erste abgedeckte Bereich einen dritten Außendurchmesser aufweist; und
eine zweite Abdeckung (132), die den zweiten Bereich (118) umgibt, wobei die zweite Abdeckung (132) und der zweite Bereich (118) einen zweiten abgedeckten Bereich bilden, und wobei der zweite abgedeckte Bereich einen vierten Außendurchmesser aufweist, wobei der dritte Außendurchmesser und der vierte Außendurchmesser im Wesentlichen gleich sind.

2. Führungsdraht (106) nach Anspruch 1, wobei die proximale Fläche (116b) des zweiten Bereichs (118) eine radiale Außenfläche des zweiten Bereichs (118) ist.

3. Führungsdraht (106) nach Anspruch 2, wobei die distale Endfläche im Wesentlichen senkrecht zur radialen Außenfläche ist.

4. Führungsdraht (106) nach Anspruch 2, wobei sich die distale Endfläche und die radiale Außenfläche an einer gekrümmten Fläche treffen.

5. Führungsdraht (106) nach Anspruch 1, wobei der mittlere Kern (113) ferner einen dritten Bereich (120) distal vom zweiten Bereich (118) aufweist, wobei der dritte Bereich (120) einen fünften Außendurchmesser aufweist, der sich vom zweiten Außendurchmesser unterscheidet.

6. Führungsdraht (106) nach Anspruch 5, wobei der mittlere Kern (113) ferner einen vierten Bereich (124) distal vom dritten Bereich (120) aufweist, wobei der vierte Bereich (124) einen sechsten Außendurchmesser aufweist, der sich vom fünften Außendurchmesser unterscheidet, und wobei der vierte Bereich (124) eine distale Spitze (142) des mittleren Kerns (113) aufweist.

7. Führungsdraht (106) nach Anspruch 1, wobei der mittlere Kern (113) ferner aufweist:
einen dritten Bereich (120) distal vom zweiten Bereich (118), wobei der dritte Bereich (120) einen fünften Außendurchmesser aufweist, der sich vom zweiten Außendurchmesser unterscheidet, und
einen vierten Bereich (124) distal vom dritten Bereich (120), wobei der vierte Bereich (124) einen sechsten Außendurchmesser aufweist, der sich vom fünften Außendurchmesser unterscheidet;
wobei der dritte Bereich (120) vom zweiten Außendurchmesser zum sechsten Außendurchmesser verjüngt ist,
wobei die zweite Abdeckung (132) mindestens einen Abschnitt des dritten Bereichs (120) umgibt,
wobei der Führungsdraht (106) ferner eine dritte Abdeckung (130) aufweist, die den vierten Bereich (124) und mindestens einen Abschnitt des dritten Bereichs (120) umgibt, wobei eine Verbindung zwischen einem am weitesten distal gelegenen Ende der zweiten Abdeckung (132) und einem am weitesten proximal gelegenen Ende der dritten Abdeckung (130) längs des verjüngten dritten Bereichs angeordnet ist.

8. Führungsdraht (106) nach Anspruch 7, wobei die zweite Abdeckung (132) eine Hülle ist.

9. Führungsdraht (106) nach Anspruch 7, wobei die zweite Abdeckung (132) aus einem ersten Material besteht, die dritte Abdeckung (130) aus einem zweiten Material besteht, das sich vom ersten Material unterscheidet, und der Führungsdraht (106) einen Ring (131) an einem Übergang (133) zwischen der zweiten Abdeckung (132) und der dritten Abdeckung (130) aufweist, wobei der Ring (131) aus einem dritten Material besteht, das sich vom ersten Material und zweiten Material unterscheidet.

10. Führungsdraht (106) nach Anspruch 7, wobei eine Wanddicke eines Abschnitts der zweiten Abdeckung (132), die den dritten Abschnitt umgibt, in eine distale Richtung abnimmt.

11. Führungsdraht (106) nach Anspruch 7, wobei der erste Bereich (114), der zweite Bereich (118) und der vierte Bereich (124) jeweils im Wesentlichen zylindrisch sind.

12. Führungsdraht (106) nach Anspruch 7, der ferner einen strahlungsundurchlässigen visuellen Indikator (138) aufweist, der auf der zweiten Abdeckung (132) aufgebracht ist, wo ein distales Ende des zweiten Bereichs (118) ein proximales Ende des dritten Bereichs (120) trifft.

13. Führungsdraht (108) nach Anspruch 7, der ferner einen strahlungsundurchlässigen visuellen Indikator (138) aufweist, der auf der dritten Abdeckung (130) aufgebracht ist.

14. Führungsdraht (106) nach Anspruch 13, wobei der strahlungsundurchlässige visuelle Indikator (138) auf der dritten Abdeckung (130) aufgebracht ist, wo ein distales Ende des dritten Bereichs (120) ein proximales Ende des vierten Bereichs (124) trifft.

## Revendications

1. Fil-guide (106) pour réaliser des procédures médicales, le fil-guide (106) comprenant :
une âme centrale (113), comprenant :
une première région (114) ayant un premier diamètre externe et une surface distale (116a), la surface distale (116a) étant une face d'extrémité distale de la première région (114), et
une deuxième région (118) à distance de la première région (114), la deuxième région (118) ayant un deuxième diamètre externe différent du premier diamètre externe et la deuxième région (118) ayant une surface proximale (116b), dans lequel au moins une partie de la surface distale (116a) de la première région (114) est coudée par rapport à une partie de la surface proximale de la deuxième région (118) ;
un premier revêtement (122) entourant la première région (114), le premier revêtement (122) et la première région (114) formant une première région recouverte, le premier revêtement (122) ayant une extrémité distale au niveau de la face d'extrémité distale de la première région (114) et la première région recouverte ayant un troisième diamètre externe ; et
un second revêtement (132) entourant la deuxième région (118), le deuxième revêtement (132) et la deuxième région (118) formant une deuxième région recouverte, et la deuxième région recouverte ayant un quatrième diamètre externe, dans lequel le troisième diamètre externe et le quatrième diamètre externe sont sensiblement égaux.

2. Fil-guide (106) selon la revendication 1, dans lequel la surface proximale (116b) de la deuxième région (118) est une surface radialement externe de la deuxième région (118).

3. Fil-guide (106) selon la revendication 2, dans lequel la face d'extrémité distale est sensiblement perpendiculaire à la surface radialement externe.

4. Fil-guide (106) selon la revendication 2, dans lequel la face d'extrémité distale et la surface radialement externe se rencontrent au niveau d'une surface incurvée.

5. Fil-guide (106) selon la revendication 1, dans lequel l'âme centrale (113) comprend en outre une troisième région (120) à distance de la deuxième région (118), la troisième région (120) ayant un cinquième diamètre externe différent du deuxième diamètre externe.

6. Fil-guide (106) selon la revendication 5, dans lequel l'âme centrale (113) comprend en outre une quatrième région (124) à distance de la troisième région (120), la quatrième région (124) ayant un sixième diamètre externe différent du cinquième diamètre externe, et la quatrième région (124) comprenant une pointe distale (142) de l'âme centrale (113).

7. Fil-guide (106) selon la revendication 1, dans lequel :
l'âme centrale (113) comprend en outre :
une troisième région (120) à distance de la deuxième région (118), la troisième région (120) ayant un cinquième diamètre externe différent du deuxième diamètre externe, et
une quatrième région (124) à distance de la troisième région (120), la quatrième région (124) ayant un sixième diamètre externe différent du cinquième diamètre externe ;
dans lequel la troisième région (120) est progressivement rétrécie du deuxième diamètre externe au sixième diamètre externe,
dans lequel le deuxième revêtement (132) entoure au moins une partie de la troisième région (120),
dans lequel le fil-guide (106) comprend en outre un troisième revêtement (130) entourant la quatrième région (124) et au moins une partie de la troisième région (120), dans lequel un joint entre l'extrémité la plus distale du deuxième revêtement (132) et l'extrémité la plus proximale du troisième revêtement (130) est positionnée le long de la troisième région progressivement rétrécie.

8. Fil-guide (106) selon la revendication 7, dans lequel le deuxième revêtement (132) est une gaine.

9. Fil-guide (106) selon la revendication 7, dans lequel le deuxième revêtement (132) est réalisé avec un premier matériau, le troisième revêtement (130) est réalisé avec un deuxième matériau différent du premier matériau, et le fil-guide (106) comprend une bague (131) au niveau d'une jonction (133) entre le deuxième revêtement (132) et le troisième revêtement (130), la bague (131) étant réalisée avec un troisième matériau différent du premier matériau et du deuxième matériau.

10. Fil-guide (106) selon la revendication 7, dans lequel une épaisseur de paroi d'une partie du deuxième revêtement (132) entourant la troisième partie diminue dans une direction distale.

11. Fil-guide (106) selon la revendication 7, dans lequel la première région (114), la deuxième région (118) et la quatrième région (124) sont chacune sensiblement cylindriques.

12. Fil-guide (106) selon la revendication 7, comprenant en outre un indicateur visuel radio-opaque (138) appliqué sur le deuxième revêtement (132) où une extrémité distale de la deuxième région (118) rencontre une extrémité proximale de la troisième région (120).

13. Fil-guide (108) selon la revendication 7, comprenant en outre un indicateur visuel radio-opaque (138) appliqué au troisième revêtement (130).

14. Fil-guide (106) selon la revendication 13, dans lequel l'indicateur visuel radio-opaque (138) est appliqué sur le troisième revêtement (130) où une extrémité distale de la troisième région (120) rencontre une extrémité proximale de la quatrième région (124).
